# EUROPEAN PATENT SPECIFICATION

(11) **EP 4 178 642 B1**
(45) Date of publication and mention of the grant of the patent: **30.04.2025**
(21) Application number: 21746568.1
(22) Date of filing: 06.07.2021
(51) Int. Cl.: A61M 1/16, A61M 1/36

(54) **CIRCUIT FOR THE EXTRACORPOREAL BLOOD CIRCULATION**
KREISLAUF FÜR DEN EXTRAKORPORALEN BLUTKREISLAUF
CIRCUIT DE CIRCULATION SANGUINE EXTRACORPORELLE

(30) Priority: 09.07.2020 IT 202000016723
(43) Date of publication of application: 17.05.2023
(73) Proprietor: Eurosets S.r.l., 41036 Medolla (MO) (IT)
(72) Inventor: PETRALIA, Antonio, 41036 Medolla (MO) (IT); GHELLI, Nicola, 41036 Medolla (MO) (IT); FONTANILI, Paolo, 41036 Medolla (MO) (IT)
(74) Representative: Zoli, Filippo
(86) International application number: PCT/IB2021/056027
(87) International publication number: WO 2022/009078

(56) References cited:
- WO-A1-00/38758
- US-A1- 2010 316 730
- US-A1- 2019 175 813

## Description

### Technical Field

The present invention relates to a circuit for the extracorporeal blood circulation.

### Background Art

By "extracorporeal blood circulation" is meant the procedure by which blood is temporarily diverted outside the patient's body to replace some of his or her vital functions.

Therefore, extracorporeal circulation can be used during certain surgical operations, during which the functions of the patient's heart are temporarily suspended and extracorporeal blood circuits are created using biomedical devices, such as the so-called "heart-lung" machines.

Extracorporeal circulation is also used during respiratory replacement therapy, during which blood decapneization is carried out aimed at providing oxygen to the blood while removing excess carbon dioxide.

There are also known therapies for blood filtration, during extracorporeal circulation, which involve the removal of waste substances that have accumulated in the blood for various reasons such as: pathological causes, surgical causes, administration of substances or other. These therapies are carried out using so-called "hemofiltration machines", which carry out the functions normally performed by healthy kidneys in proper working conditions. One such therapy is, e.g., CRRT (an acronym for Continous Renal Replacement Therapy).

The circuits for the extracorporeal blood circulation generally comprise a blood feeding line, adapted to draw the blood to be treated from the patient, a blood return line, adapted to reintroduce the treated blood into the patient and one or more biomedical devices interposed between these lines.

A blood oxygenation device is generally positioned between the blood feeding line and the blood return line which is adapted to provide the correct amount of oxygen to the patient's incoming blood and, at the same time, to remove carbon dioxide therefrom.

In particular, the oxygenation devices of known type have an inlet port of the blood to be treated, an outlet port of the treated blood, an inlet channel and an outlet channel of a working gas comprising air or oxygen and adapted to feed oxygen to the blood and/or to remove carbon dioxide from the blood itself.

Depending on the type of extracorporeal circulation carried out, the type of oxygenation device used may vary.

The circuits for the extracorporeal circulation of known type do have some drawbacks.

In fact, they may have limited effectiveness in oxygenating peripheral parts.

Another drawback of the circuits of known type consists in the difficulty to adequately oxygenate patients who, despite being sedated, have high oxygen consumption, such as, e.g., athletes.

US 2010/316730 A1 and WO 00/38758 A1 disclose circuits of the known type for the extracorporeal blood circulation comprising one oxygenation device connected at inlet to the feeding line adapted to take the blood to be treated from a patient and at outlet to the return line adapted to reintroduce the treated blood into the patient and comprising one ozone dispensing device connected to the inlet channel of a working gas into said oxygenation device for supplying oxygen to the blood to be treated and/or to remove carbon dioxide therefrom.

### Description of the Invention

The invention is defined by the features of independent claim 1.

The main aim of the present invention is to devise a circuit for the extracorporeal blood circulation which allows increasing the oxygenating capacity with respect to the circuits of known type.

Within this aim, one object of the present invention is to devise a circuit for the extracorporeal blood circulation which allows optimizing the oxygenation of the peripheral parts.

Another object of the present invention is to devise a circuit for the extracorporeal blood circulation that allows overcoming the aforementioned drawbacks of the prior art within a simple, rational, easy and effective to use as well as affordable solution.

The aforementioned objects are achieved by the present circuit for the extracorporeal blood circulation having the characteristics of claim 1.

### Brief Description of the Drawings

Other characteristics and advantages of the present invention will become more apparent from the description of a preferred, but not exclusive, embodiment of a circuit for the extracorporeal blood circulation, illustrated by way of an indicative, yet non-limiting example, in the accompanying tables of drawings wherein:

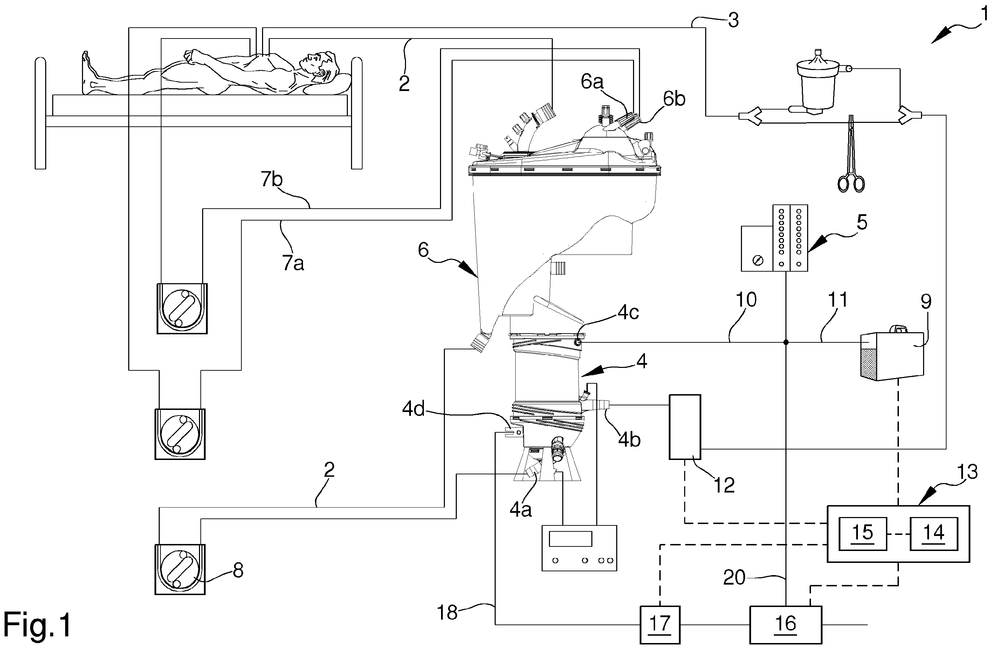

Figure 1 is a schematic representation of a circuit for the extracorporeal blood circulation according to the invention.

### Embodiments of the Invention

With particular reference to these figures, reference numeral 1 globally indicates a circuit for the extracorporeal blood circulation.

The circuit 1 comprises at least one feeding line 2 adapted to take the blood to be treated from a patient and at least one return line 3 adapted to reintroduce the treated blood into the patient.

Then, the circuit 1 comprises at least one oxygenation device 4 connected at inlet to the feeding line 2 and at outlet to the return line 3.

Such an oxygenation device 4 comprises, in turn, at least one inlet port 4a of the blood to be treated and at least one outlet port 4b of the treated blood, at least one inlet channel 4c and at least one outlet channel 4d of a working gas comprising at least one of either air or oxygen so as to supply oxygen to the blood and/or remove carbon dioxide from the blood itself.

In the present disclosure, the terms "blood to be treated" and "treated blood" mean blood arriving from the patient before it interacts with the working gas inside the oxygenation device 4 and blood being re-infused into the patient as a result of the interaction with the working gas inside the oxygenation device 4, respectively.

The circuit 1 comprises a feeding device 5 of the working gas to the oxygenation device 4 connected to the inlet channel 4c.

The feeding device 5 is therefore adapted to dispense a working gas consisting of air or oxygen, or a mixture of air and oxygen.

Preferably, as in the embodiment shown in the figures, the circuit 1 also comprises a filtering device 6 arranged along the feeding line 2, upstream of the oxygenation device 4, and adapted to filter the blood arriving from the patient. The filtering device is adapted to eliminate emboli present in the extracorporeal circuit, which can be either in gaseous or solid form.

More particularly, the filtering device 6 is provided with a first connection 6a connected to a first line 7a adapted to convey the "intracavitary" blood coming directly from the heart of the patient and a second connection 6b connected to a second line 7b adapted to convey the "extracavitary" blood.

The inlet port 4a of the oxygenation device 4 then receives the blood to be treated exiting the filtering device 6.

Conveniently, pumping means 8, e.g. of the type of a peristaltic or centrifugal pump, are positioned between the filtering device 6 and the oxygenation device 4 which are adapted to allow blood to flow in the circuit itself.

According to the invention, the circuit 1 comprises an ozone dispensing device 9 connected to the inlet channel 4c to introduce ozone into the working gas entering the oxygenation device 4. Thus, the ozone dispensing device 9 is adapted to introduce a certain amount of ozone into the working gas dispensed by the feeding device 5 and comprising air and/or oxygen.

The term "connected" as used herein means that the ozone dispensing device 9 and the inlet channel 4c are associated with each other, but not necessarily in a direct manner. In other words, by the term "connected" it is meant that the ozone dispensing device 9 may be directly associated with the inlet channel 4c but may also be placed in communication with it indirectly, that is, by interposition of other elements, as described below for the embodiment shown in the figures.

In the embodiment shown in Figure 1, the circuit 1 comprises a first channel 10 for feeding the working gas, connected on one side to the feeding device 5 and on the other side to the inlet channel 4c and at least a second channel 11 for feeding the ozone, connected on one side to the ozone dispensing device 9 and on the other side to the first feeding channel 10.

The second channel 11 engages, therefore, along the first channel 10 before the inlet channel 4c. Alternative embodiments cannot however be ruled out wherein the second channel 11 exiting the ozone dispensing device 9 is directly connected to the oxygenation device 4, and in particular to the inlet channel 4c.

According to the invention, the circuit 1 comprises sensor means 12 arranged along the return line 3 and adapted to detect the amount of ozone contained in the treated blood.

The sensor means 12 are therefore adapted to detect the amount of ozone present in the blood exiting the oxygenation device 4 after the exchange with the working gas. The treated blood exiting the oxygenation device 4 via the return line 3 is therefore enriched with oxygen and ozone compared to the blood to be treated entering the oxygenation device itself.

The value of the amount of ozone detected by the sensor means 12 is identified in the present description by the reference symbol O_{R}.

The sensor means 12 are, e.g., of the optical type.

Preferably, the sensor means 12 are of the LED type.

In more detail, the sensor means 12 may be adapted to measure the ozone concentration by detecting the absorbed wavelength or by detecting blood staining.

According to the invention, the circuit 1 comprises at least one electronic control unit 13 provided with at least one processing and command unit 14 operatively connected to the ozone dispensing device 9 and to the sensor means 12, wherein said processing and command unit 14 is programmed to intervene on the ozone dispensing device 9, so as to regulate the amount of ozone introduced into the working gas, depending on the signal received from the sensor means 12, i.e. the amount of ozone contained in the blood exiting the oxygenation device itself.

Conveniently, the electronic control unit 13 comprises at least one memory 15 programmable with at least one maximum ozone value Oₘₐₓ, and the processing and command unit 14 is programmed to compare the value of the amount of ozone O_{R} detected by the sensor means 12 with the maximum ozone value Oₘₐₓ and to intervene on the ozone dispensing device 9 in the event of the value of ozone O_{R} detected by the sensor means 12 being greater than or equal to the maximum value Oₘₐₓ so as to reduce the amount of ozone introduced into the working gas.

Alternatively, or additionally, the memory 15 may be programmable with at least one minimum ozone value Oₘᵢₙ, and the processing and command unit 14 is programmed to compare the value of the amount of ozone O_{R} detected by the sensor means 12 with the minimum ozone value Oₘᵢₙ and to intervene on the ozone dispensing device 9 in the event of the value of ozone O_{R} detected by the sensor means 12 is lower than the minimum value Oₘᵢₙ so as to increase the amount of ozone introduced into the working gas.

Preferably, the memory 15 is programmed with both the minimum value Oₘᵢₙ and the maximum value Oₘₐₓ of ozone. In this case, a range of reference ozone values Oₘᵢₙ, Oₘₐₓ is then stored, the extreme values of which are precisely defined by the minimum value Oₘᵢₙ and the maximum value Oₘₐₓ, and the processing and command unit 14 is programmed to compare the value of the amount of ozone O_{R} detected by the sensor means 12 with such a range of reference ozone values and to intervene on the ozone dispensing device 9 in the event of the value of the amount of ozone O_{R} detected by the sensor means 12 is outside the range of reference ozone values Oₘᵢₙ, Oₘₐₓ.

More particularly, the circuit 1 comprises at least one exhaust line 18 of the working gas connected to the outlet channel 4d. The working gas exiting the outlet channel 4d has a different composition from the working gas entering the oxygenation device 4 because, as anticipated above, a portion of the oxygen and ozone contained therein is transferred to the blood arriving from the patient, which in turn transfers a portion of the carbon dioxide to the working gas itself. Advantageously, the circuit 1 also comprises at least one auxiliary device 16 connected to the exhaust line 18 for the control of the working gas exiting the oxygenation device 4.

More particularly, the auxiliary device 16 is connected to at least one of the ozone dispensing device 9, the first feeding channel 10 and the second feeding channel 11 for the reintroduction of the ozone contained in the working gas leaving the oxygenation device 4 into the working gas entering the oxygenation device itself.

Conveniently, the auxiliary device 16 comprises means for the abatement of ozone (not visible in detail in the figures) contained in the working gas leaving the oxygenation device 4 and at least one exhaust port of the working gas thus abated in the environment. The function of the abatement means is to reduce the concentration of ozone in the working gas before it is released into the external environment; this is because a too high concentration of ozone in the air could be toxic to humans. More specifically, the abatement means are adapted to transform ozone, the chemical formula of which is O₃, into oxygen, i.e. O₂.

Preferably, the circuit 1 comprises auxiliary sensor means 17 positioned along the exhaust line 18 and adapted to detect the amount of residual ozone O_{L} contained in the working gas leaving the oxygenation device 4.

In more detail, the memory 15 is programmable with a value of reference residual ozone O_{LR} and the processing and command unit 14 is operationally connected to the auxiliary sensor means 17 and to the auxiliary device 16, and is programmed to intervene on the auxiliary device 16 so as to send the working gas flowing through the exhaust line 18 to the oxygenation device 4 when the amount of residual ozone O_{L} detected by the auxiliary sensor means 17 is greater than or equal to the value of reference residual ozone O_{LR} or towards the abatement means when the amount of residual ozone O_{L} detected by the auxiliary sensor means 17 is lower than the value of reference residual ozone O_{LR}.

The circuit 1 comprises a recovery line 20 of the working gas exiting the oxygenation device 4 that connects the auxiliary device 16 to one of either the first channel 10 or the second channel 11.

Advantageously, the processing and command unit 14 is operatively connected to the auxiliary sensor means 17 and is programmed to intervene on the ozone dispensing device 9 so as to regulate the amount of dispensed ozone depending on the amount of residual ozone O_{L} detected by the auxiliary sensor means 17 and reintroduced towards the oxygenation device 4.

In other words, the processing and command unit 14 is programmed to regulate the amount of ozone introduced by the ozone dispensing device 9 along the second feeding line 2 depending on the amount of residual ozone O_{L} detected by the auxiliary sensor means 17 and reintroduced into the working gas entering the oxygenation device 4.

More particularly, the greater the amount of residual ozone O_{L} present in the working gas flowing through the exhaust line 18 and the smaller the amount of ozone dispensed by the ozone dispensing device 9 and vice versa; this is in order not to fall below the amount of minimum ozone Oₘᵢₙ and not to exceed the amount of maximum ozone Oₘₐₓ set in the memory 15.

In this case, the processing and command unit 14 thus carries out a double feedback control on the ozone dispensing device 9, as it is programmed to regulate the amount of ozone dispensed depending on both the amount of ozone detected O_{R} by the sensor means and the amount of residual ozone O_{L} detected by the auxiliary sensor means 17 and recirculated in the working gas entering the oxygenation device 4.

The operation of the present invention is as follows.

The circuit 1 is adapted to take the blood to be treated from a patient and to send it towards the oxygenation device 4 via the feeding line 2.

In the embodiment shown in Figure 1, blood traveling along the feeding line 2 flows through the filtering device 6 before entering the oxygenation device 4.

At the same time, the feeding device 5 dispenses air and/or oxygen along the first feeding line 2 and the ozone dispensing device 9 dispenses a certain amount of ozone along the second feeding line 2, thus forming the working gas that is introduced inside the oxygenation device 4 through the inlet channel 4c.

The blood to be treated, once it enters the oxygenation device 4, interacts with the working gas entering through the inlet channel 4c enriching itself with oxygen and ozone while giving up carbon dioxide.

The treated blood flows out of the oxygenation device 4 through the outlet port 4b and flows along the return line 3 by which it is reinfused into the patient.

The sensor means 12 detect the amount of ozone O_{R} present in the treated blood and, by means of the processing and command unit 14 a feedback control of the amount of ozone dispensed by the ozone dispensing device 9 is carried out. In particular, the processing and command unit 14 regulates the amount of dispensed ozone, by decreasing or increasing it, depending on whether the value of detected ozone O_{R} is greater or lower than the values of maximum ozone Oₘₐₓ and the values of minimum ozone Oₘᵢₙ set in the memory 15.

At the same time, the working gas coming out of the oxygenation device 4 is conveyed along the exhaust line 18 along which the auxiliary sensor means 17 are arranged which are adapted to detect the amount of residual ozone O_{L} contained therein.

In the event of the ozone contained in the working gas flowing through the exhaust line 18 being reintroduced towards the inlet channel 4c, and thus along the first and/or the second feeding line 2, the processing and command unit 14 carries out an additional feedback control of the amount of ozone dispensed by the ozone dispensing device 9.

The processing and command unit 14 may also be programmed to reintroduce the amount of residual ozone O_{L} towards the oxygenation device 4 or towards the external environment depending on whether that amount of residual ozone O_{L} is greater or lower than a preset reference amount of ozone O_{LR}.

It has in practice been ascertained that the described invention achieves the intended objects and in particular the fact is emphasized that the circuit for the extracorporeal circulation to which the present invention relates allows, thanks to the introduction of ozone in the working gas which is introduced in the oxygenation device, increasing the oxygenating capacity of the circuit itself compared to the circuits for the extracorporeal circulation of known type.

In particular, the circuit according to the present invention allows optimizing the oxygenation of the peripheral parts during the extracorporeal blood circulation.

This allows obtaining a reactivation of microcirculation in vascular pathologies which creates an increase in the deformability of red blood cells with a consequent increase in the release of oxygen to the tissues.

Through the feedback control carried out by the processing and command unit, it is also possible to keep the amount of ozone transferred to the blood to be treated coming from the patient within optimal values, thus avoiding the occurrence of side effects due to an excess of ozone.

## Claims

1. Circuit (1) for the extracorporeal blood circulation, comprising:
- at least one feeding line (2) adapted to take the blood to be treated from a patient and at least one return line (3) adapted to reintroduce the treated blood into the patient;
- at least one oxygenation device (4) connected at inlet to said feeding line (2) and at outlet to said return line (3) and comprising at least one inlet port (4a) of the blood to be treated and at least one outlet port (4b) of the treated blood, at least one inlet channel (4c) and at least one outlet channel (4d) of a working gas comprising at least one of either air or oxygen to supply oxygen to the blood to be treated and/or to remove carbon dioxide therefrom;
- at least one feeding device (5) of the working gas connected to said inlet channel (4c);
- at least one ozone dispensing device (9) connected to said inlet channel (4c) to introduce ozone into the working gas entering said oxygenation device (4);
**characterized by** the fact that it comprises sensor means (12) arranged along said return line (3) and adapted to detect the amount of ozone (O_{R}) contained in the treated blood and
at least one electronic control unit (13) provided with at least one processing and command unit (14), operationally connected to said ozone dispensing device (9) and to said sensor means (12), said processing and command unit (14) being programmed to intervene on said ozone dispensing device (9), so as to regulate the amount of ozone introduced into the working gas, depending on the signal received from said sensor means (12).

2. Circuit (1) according to claim 1, **characterized by** the fact that it comprises a first channel (10) for feeding the working gas, connected on one side to said feeding device (5) and on the other side to said inlet channel (4c) and at least a second channel (11) for feeding the ozone, connected on one side to said ozone dispensing device (9) and on the other side to said first feeding channel (10).

3. Circuit (1) according to one or more of the preceding claims, **characterized by** the fact that said electronic control unit (13) comprises at least one memory (15) programmable with at least one maximum ozone value (Oₘₐₓ), said processing and command unit (14) being programmed to compare the value of the amount of ozone (O_{R}) detected by said sensor means (12) with said maximum ozone value (Oₘₐₓ) and to intervene on said ozone dispensing device (9) in the event of the value of ozone (O_{R}) detected by said sensor means (12) being greater than or equal to said maximum value (Oₘₐₓ) so as to reduce the amount of ozone introduced into the working gas.

4. Circuit (1) according to one or more of the preceding claims, **characterized by** the fact that said electronic control unit (13) comprises at least one memory (15) programmable with at least one minimum ozone value (Oₘᵢₙ), said processing and command unit (14) being programmed to compare the value of the amount of ozone (O_{R}) detected by said sensor means (12) with said minimum ozone value (Oₘᵢₙ) and to intervene on said ozone dispensing device (9) in the event of the value of ozone (O_{R}) detected by said sensor means (12) is lower than said minimum value (Oₘᵢₙ) so as to increase the amount of ozone introduced into the working gas.

5. Circuit (1) according to one or more of the preceding claims, **characterized by** the fact that it comprises at least one exhaust line (18) of the working gas connected to said outlet channel (4d).

6. Circuit (1) according to claim 5, **characterized by** the fact that it comprises at least one auxiliary device (16) connected to said exhaust line (18) for the control of the working gas leaving said oxygenation device (4).

7. Circuit (1) according to claim 6, **characterized by** the fact that said auxiliary device (16) is connected to at least one of either said ozone dispensing device (9), said first feeding channel (10) or said second feeding channel (11) for the reintroduction of the ozone contained in the working gas leaving said oxygenation device (4) into the working gas entering the oxygenation device itself.

8. Circuit (1) according to claim 6 or 7, **characterized by** the fact that said auxiliary device (16) comprises means for the abatement of the ozone contained in the working gas leaving said oxygenation device (4) and at least one exhaust port of the working gas thus abated in the environment.

9. Circuit (1) according to one or more of claims 5 to 8, **characterized by** the fact that it comprises auxiliary sensor means (17) positioned along said exhaust line (18) and adapted to detect the amount of residual ozone (O_{L}) contained in the working gas leaving said oxygenation device (4).

10. Circuit (1) according to claim 9, **characterized by** the fact that said processing and command unit (14) is operationally connected to said auxiliary sensor means (17) and is programmed to intervene on said ozone dispensing device (9) so as to regulate the amount of dispensed ozone depending on the amount of residual ozone (O_{L}) detected by said auxiliary sensor means (17) and reintroduced into the working gas entering said oxygenation device (4).

11. Circuit (1) according to claim 9 or 10, **characterized by** the fact that said memory (15) is programmable with a value of reference residual ozone (O_{LR}) and by the fact that said processing and command unit (14) is operationally connected to said auxiliary sensor means (17) and to said auxiliary device (16), said processing and command unit (14) being programmed to intervene on said auxiliary device (16) so as to send the working gas flowing through said exhaust line (18) to said oxygenation device (4), where the value of residual ozone (O_{L}) detected by said auxiliary sensor means (17) is greater than or equal to said value of reference residual ozone (O_{LR}), or to said abatement means when the value of residual ozone (O_{L}) detected by said auxiliary sensor means (17) is lower than said value of reference residual ozone (O_{LR}).

## Patentansprüche

1. Kreislauf (1) für den extrakorporalen Blutkreislauf, umfassend:
- mindestens eine Zufuhrleitung (2), die ausgebildet ist, um das zu behandelnde Blut einem Patienten zu entnehmen, und mindestens eine Rückführleitung (3), die ausgebildet ist, um das behandelte Blut wieder in den Patienten einzuführen;
- mindestens eine Oxygenierungsvorrichtung (4), die am Einlass mit der Zufuhrleitung (2) und am Auslass mit der Rückführleitung (3) verbunden ist und mindestens eine Einlassöffnung (4a) für das zu behandelnde Blut und mindestens eine Auslassöffnung (4b) für das behandelte Blut, mindestens einen Einlasskanal (4c) und mindestens einen Auslasskanal (4d) für ein Arbeitsgas umfasst, das Luft und/oder Sauerstoff umfasst, um dem zu behandelnden Blut Sauerstoff zuzuführen und/oder Kohlenstoffdioxid daraus zu entfernen;
- mindestens eine Zuführvorrichtung (5) für das Arbeitsgas, die mit dem Einlasskanal (4c) verbunden ist;
- mindestens eine Ozonabgabevorrichtung (9), die mit dem Einlasskanal (4c) verbunden ist, um Ozon in das Arbeitsgas einzuführen, das in die Oxygenierungsvorrichtung (4) eintritt;
**dadurch gekennzeichnet, dass** er Sensormittel (12) umfasst, die entlang der Rückführleitung (3) angeordnet und ausgebildet sind, um die im behandelten Blut enthaltene Ozonmenge (O_{R}) zu erfassen, und
mindestens eine elektronische Steuereinheit (13), die mit mindestens einer Verarbeitungs- und Befehlseinheit (14) versehen ist, die mit der Ozonabgabevorrichtung (9) und den Sensormitteln (12) in Wirkverbindung steht, wobei die Verarbeitungs- und Befehlseinheit (14) dazu programmiert ist, in die Ozonabgabevorrichtung (9) einzugreifen, um die in das Arbeitsgas eingebrachte Ozonmenge in Abhängigkeit von dem von den Sensormitteln (12) empfangenen Signal zu regulieren.

2. Kreislauf (1) gemäß Anspruch 1, **dadurch gekennzeichnet, dass** er einen ersten Kanal (10) zum Zuführen des Arbeitsgases, der auf einer Seite mit der Zuführvorrichtung (5) und auf der anderen Seite mit dem Einlasskanal (4c) verbunden ist, und mindestens einen zweiten Kanal (11) zum Zuführen des Ozons umfasst, der auf einer Seite mit der Ozonabgabevorrichtung (9) und auf der anderen Seite mit dem ersten Zuführkanal (10) verbunden ist.

3. Kreislauf (1) gemäß einem oder mehreren der vorstehenden Ansprüche, **dadurch gekennzeichnet, dass** die elektronische Steuereinheit (13) mindestens einen Speicher (15) umfasst, der mit mindestens einem maximalen Ozonwert (Oₘₐₓ) programmierbar ist, wobei die Verarbeitungs- und Befehlseinheit (14) dazu programmiert ist, den Wert der von den Sensormitteln (12) erfassten Ozonmenge (O_{R}) mit dem maximalen Ozonwert (Oₘₐₓ) zu vergleichen und auf die Ozonabgabevorrichtung (9) einzuwirken, falls der von den Sensormitteln (12) erfasste Ozonwert (O_{R}) größer oder gleich dem maximalen Wert (Oₘₐₓ) ist, um die in das Arbeitsgas eingebrachte Ozonmenge zu reduzieren.

4. Kreislauf (1) gemäß einem oder mehreren der vorstehenden Ansprüche, **dadurch gekennzeichnet, dass** die elektronische Steuereinheit (13) mindestens einen Speicher (15) umfasst, der mit mindestens einem minimalen Ozonwert (Oₘᵢₙ) programmierbar ist, wobei die Verarbeitungs- und Befehlseinheit (14) dazu programmiert ist, den Wert der von den Sensormitteln (12) erfassten Ozonmenge (O_{R}) mit dem minimalen Ozonwert (Oₘᵢₙ) zu vergleichen und auf die Ozonabgabevorrichtung (9) einzuwirken, falls der von den Sensormitteln (12) erfasste Ozonwert (O_{R}) niedriger als der minimale Wert (Oₘᵢₙ) ist, um die in das Arbeitsgas eingebrachte Ozonmenge zu erhöhen.

5. Kreislauf (1) gemäß einem oder mehreren der vorstehenden Ansprüche, **dadurch gekennzeichnet, dass** er mindestens eine Abgasleitung (18) für das Arbeitsgas umfasst, die mit dem Auslasskanal (4d) verbunden ist.

6. Kreislauf (1) gemäß Anspruch 5, **dadurch gekennzeichnet, dass** er mindestens eine mit der Abgasleitung (18) verbundene Hilfsvorrichtung (16) zur Steuerung der Ausgabe des Arbeitsgases aus der Oxygenierungsvorrichtung (4) umfasst.

7. Kreislauf (1) gemäß Anspruch 6, **dadurch gekennzeichnet, dass** die Hilfsvorrichtung (16) mit mindestens einer der folgenden Vorrichtungen verbunden ist: mit der Ozonabgabevorrichtung (9), mit dem ersten Zuführkanal (10) oder mit dem zweiten Zuführkanal (11), um das Ozon, das in dem Arbeitsgas enthalten ist, das die Oxygenierungsvorrichtung (4) verlässt, wieder in das Arbeitsgas einzuleiten, das in die Oxygenierungsvorrichtung selbst eintritt.

8. Kreislauf (1) gemäß Anspruch 6 oder 7, **dadurch gekennzeichnet, dass** die Hilfsvorrichtung (16) Mittel zur Minderung des Ozons umfasst, das in dem Arbeitsgas enthalten ist, das die Oxygenierungsvorrichtung (4) verlässt, und mindestens eine Abgasöffnung zu der Umgebung für das so geminderte Arbeitsgas.

9. Kreislauf (1) gemäß einem oder mehreren der Ansprüche 5 bis 8, **dadurch gekennzeichnet, dass** er Hilfs-Sensormittel (17) umfasst, die entlang der Abgasleitung (18) angeordnet und ausgebildet sind, um die Menge an Restozon (O_{L}) zu erfassen, die in dem Arbeitsgas enthalten ist, das die Oxygenierungsvorrichtung (4) verlässt.

10. Kreislauf (1) gemäß Anspruch 9, **dadurch gekennzeichnet, dass** die Verarbeitungs- und Befehlseinheit (14) in Wirkverbindung mit den Hilfs-Sensormitteln (17) steht und dazu programmiert ist, auf die Ozonabgabevorrichtung (9) einzuwirken, um die Menge des abgegebenen Ozons in Abhängigkeit von der Menge an Restozon (O_{L}) zu regulieren, die von den Hilfs-Sensormitteln (17) erfasst und in das in die Oxygenierungsvorrichtung (4) eintretende Arbeitsgas wieder eingeführt wird.

11. Kreislauf (1) gemäß Anspruch 9 oder 10, **dadurch gekennzeichnet, dass** der Speicher (15) mit einem Wert für das Referenz-Restozon (O_{LR}) programmierbar ist und dass die Verarbeitungs- und Befehlseinheit (14) in Wirkverbindung mit den Hilfs-Sensormitteln (17) und der Hilfsvorrichtung (16) steht, wobei die Verarbeitungs- und Befehlseinheit (14) dazu programmiert ist, auf die Hilfsvorrichtung (16) einzuwirken, um das durch die Abgasleitung (18) strömende Arbeitsgas zu der Oxygenierungsvorrichtung (4) zu leiten, wenn der von den Hilfs-Sensormitteln (17) erfasste Wert des Restozons (O_{L}) größer oder gleich dem Wert des Referenz-Restozons (O_{LR}) ist, oder zu dem Minderungsmittel, wenn der von den Hilfs-Sensormitteln (17) erfasste Wert des Restozons (O_{L}) niedriger als der Wert des Referenz-Restozons (O_{LR}) ist.

## Revendications

1. - Circuit (1) pour la circulation sanguine extracorporelle, comprenant :
- au moins une conduite d'amenée (2) apte à prélever le sang à traiter à partir d'un patient et au moins une conduite de retour (3) apte à réintroduire le sang traité dans le patient ;
- au moins un dispositif d'oxygénation (4) relié à l'entrée à ladite conduite d'amenée (2) et à la sortie à ladite conduite de retour (3) et comprenant au moins un orifice d'entrée (4a) du sang à traiter et au moins un orifice de sortie (4b) du sang traité, au moins un canal d'entrée (4c) et au moins un canal de sortie (4d) d'un gaz de travail comprenant au moins l'un parmi l'air et l'oxygène pour fournir de l'oxygène au sang à traiter et/ou pour en retirer le dioxyde de carbone ;
- au moins un dispositif (5) d'amenée du gaz de travail relié audit canal d'entrée (4c) ;
- au moins un dispositif de distribution d'ozone (9) relié audit canal d'entrée (4c) pour introduire de l'ozone dans le gaz de travail entrant dans ledit dispositif d'oxygénation (4) ;
**caractérisé par le fait qu'**il comprend des moyens capteurs (12) disposés le long de ladite conduite de retour (3) et aptes à détecter la quantité d'ozone (O_{R}) contenue dans le sang traité et
au moins une unité de commande électronique (13) comportant au moins une unité de traitement et de commande (14), reliée de manière fonctionnelle audit dispositif de distribution d'ozone (9) et auxdits moyens capteurs (12), ladite unité de traitement et de commande (14) étant programmée pour intervenir sur ledit dispositif de distribution d'ozone (9), de manière à réguler la quantité d'ozone introduite dans le gaz de travail, en fonction du signal reçu par lesdits moyens capteurs (12).

2. - Circuit (1) selon la revendication 1, **caractérisé par le fait qu'**il comprend un premier canal (10) d'amenée du gaz de travail, relié d'un côté audit dispositif d'amenée (5) et de l'autre côté audit canal d'entrée (4c) et au moins un second canal (11) d'amenée de l'ozone, relié d'un côté audit dispositif de distribution d'ozone (9) et de l'autre côté audit premier canal d'amenée (10) .

3. - Circuit (1) selon une ou plusieurs des revendications précédentes, **caractérisé par le fait que** ladite unité de commande électronique (13) comprend au moins une mémoire (15) programmable avec au moins une valeur maximale d'ozone (Oₘₐₓ), ladite unité de traitement et de commande (14) étant programmée pour comparer la valeur de la quantité d'ozone (O_{R}) détectée par lesdits moyens capteurs (12) à ladite valeur maximale d'ozone (Oₘₐₓ) et pour intervenir sur ledit dispositif de distribution d'ozone (9) dans le cas où la valeur d'ozone (O_{R}) détectée par lesdits moyens capteurs (12) est supérieure ou égale à ladite valeur maximale (Oₘₐₓ) de manière à réduire la quantité d'ozone introduite dans le gaz de travail.

4. - Circuit (1) selon une ou plusieurs des revendications précédentes, **caractérisé par le fait que** ladite unité de commande électronique (13) comprend au moins une mémoire (15) programmable avec au moins une valeur minimale d'ozone (Oₘᵢₙ), ladite unité de traitement et de commande (14) étant programmée pour comparer la valeur de la quantité d'ozone (O_{R}) détectée par lesdits moyens capteurs (12) à ladite valeur minimale d'ozone (Oₘᵢₙ) et pour intervenir sur ledit dispositif de distribution d'ozone (9) dans le cas où la valeur d'ozone (O_{R}) détectée par lesdits moyens capteurs (12) est inférieure à ladite valeur minimale (Oₘᵢₙ) de manière à augmenter la quantité d'ozone introduite dans le gaz de travail.

5. - Circuit (1) selon une ou plusieurs des revendications précédentes, **caractérisé par le fait qu'**il comprend au moins une conduite d'échappement (18) du gaz de travail reliée audit canal de sortie (4d).

6. - Circuit (1) selon la revendication 5, **caractérisé par le fait qu'**il comprend au moins un dispositif auxiliaire (16) relié à ladite conduite d'échappement (18) pour le contrôle du gaz de travail sortant dudit dispositif d'oxygénation (4).

7. - Circuit (1) selon la revendication 6, **caractérisé par le fait que** ledit dispositif auxiliaire (16) est relié à au moins l'un dudit dispositif de distribution d'ozone (9), dudit premier canal d'amenée (10) et dudit second canal d'amenée (11) pour la réintroduction de l'ozone contenu dans le gaz de travail sortant dudit dispositif d'oxygénation (4) dans le gaz de travail entrant dans le dispositif d'oxygénation lui-même.

8. - Circuit (1) selon l'une des revendications 6 ou 7, **caractérisé par le fait que** ledit dispositif auxiliaire (16) comprend des moyens d'abattement de l'ozone contenu dans le gaz de travail sortant dudit dispositif d'oxygénation (4) et au moins un orifice d'échappement du gaz de travail ainsi abattu dans l'environnement.

9. - Circuit (1) selon une ou plusieurs des revendications 5 à 8, **caractérisé par le fait qu'**il comprend des moyens capteurs auxiliaires (17) disposés le long de ladite conduite d'échappement (18) et aptes à détecter la quantité d'ozone résiduel (O_{L}) contenue dans le gaz de travail sortant dudit dispositif d'oxygénation (4).

10. - Circuit (1) selon la revendication 9, **caractérisé par le fait que** ladite unité de traitement et de commande (14) est reliée de manière fonctionnelle auxdits moyens capteurs auxiliaires (17) et est programmée pour intervenir sur ledit dispositif de distribution d'ozone (9) de manière à réguler la quantité d'ozone distribué en fonction de la quantité d'ozone résiduel (O_{L}) détectée par lesdits moyens capteurs auxiliaires (17) et réintroduite dans le gaz de travail entrant dans ledit dispositif d'oxygénation (4).

11. - Circuit (1) selon l'une des revendications 9 ou 10, **caractérisé par le fait que** ladite mémoire (15) est programmable avec une valeur d'ozone résiduel de référence (O_{LR}) et **par le fait que** ladite unité de traitement et de commande (14) est reliée de manière fonctionnelle auxdits moyens capteurs auxiliaires (17) et audit dispositif auxiliaire (16), ladite unité de traitement et de commande (14) étant programmée pour intervenir sur ledit dispositif auxiliaire (16) de manière à envoyer le gaz de travail s'écoulant à travers ladite conduite d'échappement (18) audit dispositif d'oxygénation (4), lorsque la valeur d'ozone résiduel (O_{L}) détectée par lesdits moyens capteurs auxiliaires (17) est supérieure ou égale à ladite valeur d'ozone résiduel de référence (O_{LR}), ou auxdits moyens d'abattement lorsque la valeur d'ozone résiduel (O_{L}) détectée par lesdits moyens capteurs auxiliaires (17) est inférieure à ladite valeur d'ozone résiduel de référence (OLR) .
